# EUROPEAN PATENT APPLICATION

(11) **EP 1 350 512 A1**
(43) Date of publication of application: **08.10.2003**
(21) Application number: 02007568.5
(22) Date of filing: 03.04.2002
(51) Int. Cl.: A61K 31/46, A61P 37/08

(54) **Combinations of epinastine, belladonna and pseudoephedrine as new pharmaceutical formulations**

(71) Applicant: Boehringer Ingelheim International GmbH, 55216 Ingelheim am Rhein (DE)
(72) Inventor: Seko, Noritaka, Osaka 563-0028 (JP)

(57) **Abstract**

The present invention relates to novel oral pharmaceutical compositions comprising as pharmaceutically active compounds a combination of an antihistaminic-effective amount of epinastine or a pharmaceutically acceptable salt thereof, an anticholinergic amount of Belladonna alkaloids (Belladonna) or a pharmaceutically acceptable salt thereof and of a decongestant-effective amount of pseudoephedrine or a pharmaceutically acceptable salt thereof. Optionally, the formulation may comprise methylephedrine (methylephrine) in a decongestant-effective amount or a pharmaceutically acceptable salt thereof. The formulation further comprises suitable pharmaceutically acceptable carriers or excipients. Another aspect of the present invention relates to methods for the preparation of these compositions and methods of using them in the treatment of allergic diseases and/or disorders. In particular the inventive composition is useful in the treatment of saisonal allergic rhinitis and saisonal allergic conjunctivitis.

## Description

The present invention relates to novel oral pharmaceutical compositions comprising as pharmaceutically active compounds a combination of an antihistaminic-effective amount of epinastine or a pharmaceutically acceptable salt thereof, an anticholinergic amount of Belladonna alkaloids (Belladonna) or a pharmaceutically acceptable salt thereof and of a decongestant-effective amount of pseudoephedrine or a pharmaceutically acceptable salt thereof. Optionally, the formulation may comprise methylephedrine (methylephrine) or a pharmaceutically acceptable salt thereof in a decongestant-effective amount. The formulation further comprises suitable pharmaceutically acceptable carriers or excipients. Another aspect of the present invention relates to methods for the preparation of these compositions and methods of using them in the treatment of allergic diseases and/or disorders. In particular the inventive composition is useful in the treatment of saisonal allergic rhinitis and saisonal allergic conjunctivitis.

### Background of the invention

Saisonal allergic rhinitis (SAR) and saisonal allergic conjunctivitis (SAC) are allergic driven diseases with a specific symptomatology.

SAR is characterised by sneezing, itching, blocked nose ("congested nose") and runny nose, while SAC is characterised by eye itching, red eye and sensation of foreign body. Both allergic reactions may occur separately of each other or at the same time.

An adequate systemic symptomatological treatment of SAR and SAC should address all the symptoms.

From the state of the art there is not known any suitable substance able to deal with all the symptoms.

It is known that H1 antihistamine will deal with the histamine driven symptoms such as sneezing or itching. H1 antihistamine may also have an effect on runny noses or red eyes but to a lesser grade. Due to this fact they are not the first choice substances to treat the latter. Additionally, H1 antihistamines are unable treat blocked noses.

From JP-A 10298107 it is known that hyperergasis of respiratory tract secretion may be suppressed by compositions comprising H1 antihistamines and anticholinerics.

However, the aforementioned combination is only useful to treat some of the symptoms related to allergic disorders as SAR or SAC.

In particular these formulations or combinations are not suited to treat the issue of blocked noses or red eyes.

It now was found that the combination of epinastine, an H1 antihistaminic agent, Belladonna alkaloids and pseudoephedrine successfully treat all the aforementioned symptoms of SAR or SAC.

### Objective of the present invention

It is one objective of the present invention to treat the symptoms of seasonal allergic rhinitis, i.e. sneezing, itching, blocked nose and runny nose.

It is another objective of the present invention to treat the symptoms of seasonal conjunctivitis, i.e. eye itching, red eyes and sensation of foreign bodies.

It is another objective to develop one medication to treat the symptoms of both diseases, SAR and SAC simultaneously.

Another objective is to develop a suitable pharmaceutical formulation for treating allergic congestion of the Eustachian tubes and / or other diseases from allergic origin deserving the administration of antihistamine and decongestant drugs.

Another objective of the present invention is the treatment of common cold and in the symptomatic relief associated with cough, cold and flu symptoms.

Still another objective of the present invention is to overcome the disadvantages of the medications known in the art in the treatment of SAR and/or SAC.

Among theses objectives, the developments of medications to treat SAR and/or SAC are preferred.

### Description of the invention

The present invention solves the problem of insufficient treatment of SAR and/or SAC by providing a pharmaceutical formulation comprising an antihistaminic-effective amount of epinastine or a pharmaceutically acceptable salt thereof, an anticholinergic amount of Belladonna alkaloids or a pharmaceutically acceptable salt thereof and of a decongestant-effective amount of pseudoephedrine or a pharmaceutically acceptable salt thereof. Optionally, the formulation may additionally comprise methylephedrine or a pharmaceutically acceptable salt thereof in a decongestant-effective amount. Further ingredients of the formulation of the present invention may be pharmaceutically acceptable carriers or excipients.

The term Belladonna alkaloids is commonly used in pharmaceutics. The exact method of their winning and the active ingredients of this mixture of alkaloids can be taken from the Deutsches Arzneibuch 9 (DAB 9), Volume 2, pages 932 to 944, Wissenschaftliche Verlagsgesellschaft Stuttgart mbH; Govi-Verlag GmbH, Frankfurt. These pages 932 to 944 are herewith incorporated by reference. Belladonna alkaloids are won as an extract of the plant Atropa Belladonna, i.e. an extracts of the leafs and/or the root.
The main component of the Belladonna alkaloids is atropin. Atropine itself comprises L-(-)-hyoscyamine and its racemate which develops by drying. Other alkaloids found in Belladonna are L-(-)-hyoscine (L-(-)-scopolamine), N-Oxides of hyoscine and/or hyoscamine, atropamine, belladonnine and optionally nicotine, N-methylpyrroline, N-methylpyrrolidine, pyridin, cuskhygrine and further alkaloids. The names of the alkaloids as written above are taken from the German textbook DAB 9, referred to above. In case of ambiguities the names shall be taken directly from the textbook, page 934.

Preferably in the context of the present invention the mixture of the above named alkaloids are taken. However, the invention is not limited to the use of this exact mixture. In fact, any mixture or any single alkaloid of the designated alkaloids extracted from Atropa Belladonna can be used. In particular, the invention comprises atropin or L-(-)-hyoscyamin alone without the other named alkaloids. In the context of the present invention, the term belladonna alkaloids preferably stands mainly for hyoscyamin and scopolamine as major components in extract of belladonna roots and/or leaves. These anticholinergic alkaloids have analgesic-antispasmodic action and inhibitory action of secretion. Extract of Datura can also be selected as a substitute for belladonna alkaloids.

Also the above mentioned active ingredients are the preferred ones and as a consequence thereof the formulation preferably does not contain any further active ingredients, the formulation of the present invention is not limited to theses active ingredients alone. As an additional active compound the compositions according to the invention may optionally contain one or several compounds selected from the group consisting of mucolitic and analgesic-antipyretic compounds and vitamins. Preferred mucolitic ingredients are selected from bromhexine and ambroxol. Preferred analgesic-antipyretic compounds are selected from paracetamol and ibuprofen. Preferred vitamins are selected from vitamin B2. B6 and C. Preferably a leukotriene antagonist is not present.

In a preferred embodiment the present invention relates to an oral pharmaceutical composition. Due to the short-lasting effects of pseudoephedrine and Belladonna and - relatively to this - the long-lasting effect of epinastine it is of advantage to have a sustained release of Belladonna and the decongestant effective amount of pseudoephedrine and/or methylephrine and an immediate release of an antihistaminic effective amount of epinastine.

The preferred dosage forms are tablets or capsules.

Concerning the application via a tablet, in the context of the present the invention a bilayer tablet is preferred wherein a first layer A provides for the sustained release of Belladonna and pseudoephedrine, which comprises a decongestant effective amount of pseudoephedrine or a pharmaceutically acceptable salt thereof and a anticholinergic amount of Belladonna or a pharmaceutically acceptable salt thereof. A second layer B provides for the immediate release of epinastine and comprises an antihistaminic effective amount of epinastine or a pharmaceutically acceptable salt thereof. In case the formulation contains additionally methylephedrine or one of its pharmaceutically acceptable salts, the appropriate amount thereof is present in layer A, already comprising pseudoephedrine.

Both layers A or B may further comprise pharmaceutically acceptable excipients and/or carriers.

The bilayer tablet according to the invention may additionally contain a tablet coating C consisting of pharmaceutically acceptable excipients, which mask the bitter taste of one of the active compounds.

In a preferred embodiment of the inventive bilayer tablet layer A comprises a decongestant effective amount of pseudoephedrine or a pharmaceutically acceptable salt thereof and optionally methylephedrine or a pharmaceutical acceptable salt thereof in a matrix of a swellable hydrophilic polymer which provides a sustained release profile in a period of 3 to 24, preferably 6 to 18, most preferably about 12 hours.

In another application form the inventive composition may be formulated as a capsule. Such a capsule can provide the active ingredients either instantly or some of them are provided instantly and others are provided in a sustained manner. As outlined above it is preferred to formulate the active ingredients pseudoephedrine (or its salts) and Belladonna alkaloids (or its salts thereof) as well as the optionally used methylephrine (or its salts) as a sustained releases form and epinastine or its salts as immediate release form.

Preferably the capsules are made of materials that at least partially can be digested by humans. Such capsules f.e. are disclosed in EP 0143524. The latter discloses a two-part capsule of material which is easily digestible by humans.

EP 0460921 describes capsules of chitosan and starch, grain powder, oligosaccharides, methacrylic acid-methylacrylate, methacrylic acid-ethylacrylate, hydroxypropylmethylcelluloseacetate, -succinate or -phthaleate.

GB 938828 discloses capsules comprising water-soluble gelatine, methylcellulose, Polyvinylalkohol or water-soluble non-toxic thermoplasts.

EP 0 606 486 B1 discloses capsules being composed of hydroxypropylmethylcellulose, methylcellulose, hydroxypropylcellulose, starch, hydroxypropylstarch, and sodium alginate.

Principally all theses capsules can be take for the present invention, preferred are gelatine-capsules, in particular hard-gelatine capsules. Other preferred capsules are made of starch or of a cellulose-derivative like hydroxy-propylmethylcellulose.

Preferred standard capsules have the following physical characteristics:

| Size | 5 | 4 | 3 | 2 | 1 | 0 |
|---|---|---|---|---|---|---|
| Filling-weight [mg] | 65 | 100 | 150 | 185 | 250 | 340 |
| | | | | | | |

| Outside-Diameter | | | | | | |
|---|---|---|---|---|---|---|
| [mm] | | | | | | |
| Cap | 4.89 | 5.31 | 5.82 | 6.35 | 6.90 | 7.63 |
| Body | 4.66 | 5.06 | 5.56 | 6.07 | 6.61 | 7.32 |
| | | | | | | |

| Length [mm] | | | | | | |
|---|---|---|---|---|---|---|
| (± 0.3mm) | | | | | | |
| Cap | 6.05 | 7.47 | 8.23 | 9.17 | 10.01 | 11.18 |
| Body | 9.40 | 12.34 | 13.61 | 15.24 | 16.71 | 18.72 |
| | 0.13 | 0.21 | 0.28 | 0.37 | 0.49 | 0.68 |
| | | | | | | |

| Body-Volume [ml] | | | | | | |
|---|---|---|---|---|---|---|
| Capsules-weight [mg] (±10%) | 28.1 | 40.0 | 50.7 | 65.2 | 76.0 | 99.0 |

Among them capsule-size of 1 or 2 are preferred.

According to the invention the term pharmaceutically acceptable salts stands for acid addition salts of the active compounds pseudoephedrine, epinastine, Belladonna alkaloids and/or methylephedrine. These acid addition salts can be formed with inorganic acids like hydrochloric acid, hydrobromic acid or sulfuric acid or with organic acids as for instance oxalic acid, fumaric acid or methansulfonic acid. Epinastine is preferably used as its hydrochloric acid addition salt. Pseudoephedrine and also methylephedrine are preferably used as the hydrochlorides or the sulfates. Within the present invention the hydrochloride-salts for the latter two compounds are most preferred.

The release of pseudoephedrine and optionally methylephedrine takes place over 3 to 24, preferably 6 to 24, most preferably about 12 to 24 hours. The preferred dose regimen is a "once a day application", nevertheless how the formulation is applied.

The concentration range of pseudoephedrine salt plus methylephedrine salt in the compositions according to the invention is between 5 and 240 mg , preferably 10 to 200 mg, more preferably 20 to 150 mg, preferably 50 to 130 mg, most preferably 60. 78 or 120 mg.
The composition is preferably such, that the amount of pseudoephedrine does not exceed 10% w/w of the total amount of the composition.
Furthermore, preferably the amount of pseudoephedrine in the formulation is such, that the total amount delivered each day does not exceed 60 mg. To reach this objective it might be of advantage to reduce the amount of pseudoephedrine and as a consequence thereof the amount of the other active ingredients as well.

If methylephedrine or a salt thereof is present, what is preferred, both compounds pseudoephedrine and methylephedrine are preferably present in the formulation in the same amount, i.e. amount w/w. Thus, to reach a total amount of pseudoephedrine plus methylephedrine (their salts respectively) of f.e. 78 mg each of the two compounds is present in an amount of 39 mg, for a total amount of 60 mg, each compound is present in an amount of 30 mg.

The concentration range of epinastine salt in the compositions according to the invention is between 2 and 20 mg, preferably 5 to 10 mg, more preferably 10 mg daily.

The concentration range of Belladonna alkaloids in the compositions according to the invention is between 0.05 and 4.0 mg, preferably 0.1 to 1.5 mg, more preferably between 0.2 and 0.6 mg.

In case of a bilayer tablet, each layer is in contact with each other in a portion of their surface, but provides independent release profiles for both active substances mentioned before.
The sustained release layer A comprises beside the active ingredient(s) a swellable hydrophilic polymer.
Typical swellable hydrophilic polymers include cellulose ethers such as methylcellulose, hydroxypropylcellulose, hydroxypropylmethylcellulose, hydroxymethylcellulose, hydroxyethylcellulose, carboxymethylcellulose and carboxyethylcellulose or mixtures thereof. The use of hydroxypropylmethylcellulose (HPMC) is preferred. Particularly useful are the HPMC polymers HPMC USP2910 and USP2208 like for instance Methocel E5. E4M, E15M, K15M, and K100M supplied by the Dow Chemical Company. In the aforementioned abbreviations the designation "E" refers to USP2910 whereas "K" refers to USP2208. The number designation refers to the viscosity in a 2% aqueous solution (e.g. 5 designates a viscosity of 5 cps; 15M designates a viscosity of 15000 cps).

The excipients that could be optionally used in the sustained release layer A are insoluble polymers, soluble or insoluble fillers, antiadherents, coloring agents, lubricants and additional binders. Typical fillers are for example lactose, microcrystalline cellulose, dibasic calcium phosphate and cornstarch. Examples of antiadherents, which are used to prevent tablets from sticking to the tablet press, are colloidal silicon dioxide and talc. Magnesium stearate, talc and stearic acid are typical lubricants. Typical binders are povidone, and cornstarch.

The immediate release matrix layer B comprises beside the active ingredient different combinations of excipients. The excipients that could be optionally used in the immediate release layer B are insoluble polymers, soluble or insoluble fillers, antiadherents, lubricants, coloring agents, disintegrants and additional binders.
Typical fillers are for example lactose, microcrystalline cellulose, dibasic calcium phosphate and cornstarch. Examples of antiadherents, which are used to prevent tablets from sticking to the tablet press, are colloidal silicon dioxide and talc. Typical disintegrants are crospovidone, sodium starch glycolate and crosscarmellose sodium. Typical coloring agents are selected from FD&C red 40 HT Aluminum lake, 2-hydroxy-1.1'-azonaphthalene-3.6.4'-trisulfonic acid trisodium salt, erythrosine, iron oxides, 1-(4-sulpho-1-naphthylazo)-2-naphthol-6.8-disulphonic acid trisodium salt, 2',4',5',7'-tetrabromo-4.5.6.7-tetrachloro-fluorescein disodium salt, 2.4.5.7-Tetraiodo-3.6-dihydroxyxanthene-9-spiro-1'-(4',5',6',7'-tetrachloro-3'H-isobenzofuran-3'one dipotassium salt, trisodium 3-carboxy-5-hydroxy-1-p-sulphophenyl-4-p-sulfophenylazopyrazole, 6-hydroxy-5-((4-sulphonphenyl)azo-2-naphthalenesulphonic acid disodium salt and optionally aluminium lakes thereof. Magnesium stearate, talc and stearic acid are typical lubricants. Typical binders are povidone, and cornstarch.

Water and ethanol are examples of volatile components which can be used in the manufacture process of both layers to granulate powders. These volatile components are removed during processing and therefore do not appear in the finished product.

The tablet coating is optional since the presence of it does not modifies significantly the release rates of the active substances present in the core layers. The presence of the coating is preferred because it masks the bitter taste of one of the active substances and enhances the properties of dosage form. Because of that a lot different coatings with different polymers, and plasticizers and other excipients could be used with the condition of not modifying significantly the release profile of the active substances present in the core tablet. A typical coating comprises a polymer such as hydroxypropylmethylcellulose and a plasticizer such as polyethylene glycol. Optional excipients could be added to the coating like antifoaming agents and opacifying agents. Example of an antifoaming agent is silicone. Examples of opacifying agents are Titanium dioxide, talc and aluminum lake dyes.

The inventive formulation also can be applied via a tablet comprising sustained release and non-sustained release granules or a capsule comprising the same.

In case of such a tablet, non-sustained release granules and sustained release granules, which are coated with a sustained release film are mixed with suitable excipients and then they are compressed as a tablet.

Similarly non-sustained release granules and sustained release granules which are coated with sustained release film film are mixed and filled into a capsule.

A non-sustained release granule comprises an amount of epinastine or a pharmaceutically acceptable salt thereof. Optionally it may comprises a portion of the total amount of belladonna alkaloids or a pharmaceutically acceptable salt thereof and a portion of the total amount of pseudoephedrine or a pharmaceutically acceptable salt thereof and optionally a portion of the total amount of methylephedrine or a pharmaceutically acceptable salt thereof, if necessary.

A sustained release granule comprises either a portion or the total amount of belladonna alkaloids or a pharmaceutically acceptable salt thereof, pseudoephedrine or a pharmaceutically acceptable salt thereof and optionally methylephedrine or a pharmaceutically acceptable salt thereof.

Preferably the non-sustained release granules contain only epinastine or a pharmaceutically acceptable salt thereof as active ingredient while the the sustained release granules comprise the remaining active ingedients.

Any compounds conventionally used as a sustained-release coat can be used for the purpose of this invention. Specific examples which can be given include water insoluble polymers such as ethylcellulose, aminoalkyl methacrylate copolymer polyvinyl acetate, polyvinyl chloride, polyethylene, and the like; intestinally soluble polymers such as cellulose acetate phthalate, hydroxypropyl methylcellulose phtharate, hydroxypropyl methylcellulose acetate succinate, carboxymethylethylcellulose, styrene acrylic acid copolymer, methacrylic acid copolymer, maleic anhydrous acid copolymer, shellac, and the like; paraffin waxes such as paraffin, microcrystalline wax, and the like; higher alcohols, preferably saturated and unsaturated C₆ - C₂₆-alcohols, preferred unbranched and unsubstituted, such as stearyl alcohol, cetyl alcohol, and the like; esters of higher fatty acids, preferably saturated and unsaturated C₆ - C₂₆-acids, preferred unbranched and unsubstituted, such as glycerine fatty acid esters, hydrogenated oils, carnauba wax, beeswax, Japan (haze) wax, and the like; and higher fatty acids as defined above such as stearic acid, palmitic acid, myristic acid, behenic acid, and the like (or the sodium, calcium or magnesium salts of these higher fatty acids).

Furthermore, the excipients that could be optionally used in sustained release film are water soluble polymers, sugar alcohols, plasticizers, titanium oxide, talc, coloring agents and so on. Typical water soluble polymers and sugar alcohols are hydroxypropyl methylcellulose, hydroxypropylcellulose, methylcellulose, polyvinylpyrrolidone, polyethylene glycol. Typical plasticizers are glycerine fatty acid ester, triethyl citrate, propylene glycol, triacetin.

For any of the inventive application forms, bilayer tablet, tablet or capsule any of the aformentioned ingredients can be taken, if appropriate.

In the context of the present invention capsules and tablets comprising sustained release and non-sustained release granules are preferred.

The invention will be further described by the following examples. These examples disclose certain preferred embodiments of the invention. The methods of manufacturing the compositions according to the invention like for instance granulation, tablet compression, tablet-coating etc. are well known to the person skilled in the art. Those skilled in the art will appreciate that various changes, modifications and substitutions can be made therein without departing from the spirit of the invention. Accordingly, it is intended that the invention be not limited to the following explicitly disclosed examples.

**Example N°1 -** Composition

### Core

| **A. First layer** | |
|---|---|
| **Layer pseudoephedrine, Belladonna, Methylephedrine** | **mg/tablet** |
| Pseudoephedrine hydrochloride | 30.00 |
| Methylephedrine hydrochloride | 30.00 |
| Belladonna | 0.15 |
| Methocel K 15 M PRCR* | 99.00 |
| Lactose Monohydrate | 52.4 |
| Microcrystalline cellulose | 53.00 |
| Colloidal silicon dioxide | 0.825 |
| Magnesium Stearate | 1.375 |
| Povidone | 8.25 |
| Total first layer | 275.00 |

| **B. Second layer** | |
|---|---|
| **Layer Epinastine** | **mg**/**tablet** |
| Epinastine HCI | 5.00 |
| FD&C red 40 HT Aluminum lake (allura red AC) | 0.19 |
| Microcrystalline cellulose | 35.00 |
| Lactose Monohydrate | 77.31 |
| Povidone | 6.25 |
| Magnesium Stearate | 1.25 |
| Total second layer | **125.00** |
| | |
| **Total core** | **400.00** |

| **C. Coating** | |
|---|---|
| **Film Coating** | **mg/ tablet** |
| Methocel E5 | 7.50 |
| Polyethylene Glycol 6000 | 0.985 |
| Silicone antifoam S184 | 0.015 |
| Total film coating | **8.50** |
| | |
| **Total Film coated tablet** | **408.50** |

| | |
|---|---|
| * PR means Premium grade and CR means Controlled Released grade. | |

### Method of Manufacture

### A. First layer:

A1. Dissolve povidone in a hydroalcoholic mixture;
A2. Blend pseudoephedrine hydrochloride, methylephedrine hydrochloride, Belladonna alkaloids, a portion of the microcrystalline cellulose, lactose and Methocel K15M for 5-30 minutes in a suitable mixer.
A3. Use alcoholic or hydroalcoholic solution prepared previously in step A1 to granulate the powder mix of step A2.
A4. Dry and mill the granulation from step A3, using suitable size screen.
A5. Blend the screened granulation with a portion of the microcrystalline cellulose and colloidal silicon dioxide for 3-15 minutes.
A6. Add magnesium stearate and blend for 3-15 minutes.

### B Second layer:

B1. Pass through a suitable screen Epinastine HCL, Allura red AC (FD & C red 40 HT) aluminum lake and microcrystalline cellulose. Blend for 5-30 minutes in a suitable mixer.
B2. Add lactose and povidone. Blend for 60 minutes 15-120 minutes in a suitable mixer.
B3. Add magnesium stearate. Blend for 3-20 minutes in a suitable mixer.

### C. Compression:

Compress A and B into a suitable bilayer tableting machine in suitable size tablets.

### D. Coating

D1. Dissolve Methocel E5 and Polyethylene Glycol in suitable amount of water. D2. Dissolve silicone antifoam in suitable amount of isopropilic alcohol.
D3. Add 2. to 1. and mix.
D4. Coat tablets with the Methocel E5 /Polyethylene glycol solution from step D3. in a suitable coater.

### Example N° 2 - Composition

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K 15 M PRCR * | 99.00 |
| Lactose Monohydrate | 63.10 |
| Microcrystalline cellulose | 50.00 |
| Colloidal silicon dioxide | 1.375 |
| Magnesium Stearate | 1.375 |
| Total first layer | 225.00 |

| **B. Second layer** | |
|---|---|
| **Layer Epinastine** | **Mg/tablet** |
| Epinastine HCI | 5.00 |
| Lactose Monohydrate | 84.20 |
| Microcrystalline cellulose | 35.00 |
| Punceau 4R red aluminum lake | 0.175 |
| Magnesium Stearate | 0.625 |
| Total second layer | **125.00** |
| | |
| **Total core** | **400.00** |

| **C. Coating** | |
|---|---|
| **Film Coating** | **mg/ tablet** |
| Methocel E5 | 2.21 |
| Polyethylene Glycol 6000 | 1.36 |
| Talc | 4.38 |
| Titanium dioxide | 0.55 |
| Total film coating | 8.50 |
| | |
| **Total Film coated tablet** | **408.50** |

| | |
|---|---|
| * PR means Premium grade and CR means Controlled Released grade. | |

### Method of Manufacture

### A. First layer:

A1. Blend pseudoephedrine hydrochloride, methylephedrine hydrochloride, Belladonna alkaloids, microcrystalline cellulose, lactose, colloidal silicon dioxide and HPMC K15M for 5-30 minutes in a suitable mixer.
A2. Add magnesium stearate and blend for 3-15 minutes.

### B. Second layer:

B1. Pass through a suitable screen Epinastine HCI, and microcrystalline cellulose. Blend for 5-30 minutes in a suitable mixer.
B2. Add lactose. Blend for 60 minutes 15-120 minutes in a suitable mixer.
B3. Add magnesium stearate. Blend for 3-20 minutes in a suitable mixer.

### C. Compression:

Compress A and B into a suitable bilayer tableting machine in suitable size tablets.

### D. Coating

D1. Dissolve Methocel E5 and Polyethylene Glycol in suitable amount of water.
D2. Add Titanium Dioxide and Talc in suitable amount of water and mix
D3. Add 2. to 1. And mix.
D4. Coat tablets with the Methocel E5 /Polyethylene glycol solution from step D3. in a suitable coater.

### Example N° 3

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K4M PRCR | 123.75 |
| Lactose Monohydrate | 82.85 |
| Talc | 5.5 |
| Magnesium Stearate | 2.75 |
| **Total first layer** | **275.00** |
| * PR means Premium grade and CR means Controlled Released grade. | |

Second layer and coating are identical to example 2; the manufacture method was conducted analogously to the method outlined in example 2;

### Example N° 4

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15 M PRCR | 99.00 |
| Lactose Monohydrate | 49.60 |
| Microcrystalline cellulose | 49.75 |
| Colloidal silicon dioxide | 1.375 |
| Povidone | 13.75 |
| Magnesium stearate | 1.375 |
| **Total** | **275.0 0** |
| * PR means Premium grade and CR means Controlled Released grade. | |

Second layer and coating are identical to example 1; the manufacture method was conducted analogously to the method outlined in example 1;

### Example N° 5

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 165.00 |
| Lactose | 41.6 |
| Talc | 5.50 |
| Magnesium Stearate | 2.75 |
| **Total** | **275.00** |
| * CR means Controlled Released grade. | |

Second layer and coating are identical to example 1; the manufacture method was conducted analogously to the method outlined in example 1;

### Example N° 6

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 137.50 |
| Microcrystalline Cellulose | 69.10 |
| Talc | 5.50 |
| Magnesium Stearate | 2.75 |
| Ethanol | sq. |
| **Total** | **275.00** |
| * CR means Controlled Released grade. | |

Second layer and coating are identical to example 1; the manufacture method was conducted analogously to the method outlined in example 1;

### Example N° 7

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 107.50 |
| Dibasic Calcium phosphate | 54.10 |
| Ethylcelullose | 20.00 |
| Talc | 5.50 |
| Magnesium Stearate | 2.75 |
| Ethanol | s.q. |
| **Total** | **250.00** |
| * CR means Controlled Released grade. | |

Second layer and coating are identical to example 1; the manufacture method was conducted analogously to the method outlined in example 1;

### Example N° 8

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 68.75 |
| Methocel K100M CR | 68.75 |
| Lactose | 69.10 |
| Talc | 5.50 |
| Magnesium Stearate | 2.75 |
| Ethanol | s.q. |
| **Total** | **275.00** |
| * CR means Controlled Released grade. | |

Second layer and coating are identical to example 1; the manufacture method was conducted analogously to the method outlined in example 1;

### Example N° 9

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K100M CR | 137.50 |
| Lactose | 69.1 |
| Talc | 5.50 |
| Magnesium Stearate | 2.75 |
| Ethanol | s.q. |
| **Total** | **275.00** |
| * CR means Controlled Released grade. | |

Second layer and coating are identical to example 1; the manufacture method was conducted analogously to the method outlined in example 1;

### Example N° 10

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 103.10 |
| Methocel K100M CR | 34.40 |
| Lactose | 69.10 |
| Talc | 5.50 |
| Magnesium Stearate | 2.75 |
| Ethanol | s.q. |
| **Total** | **275.00** |
| * CR means Controlled Released grade. | |

Second layer and coating are identical to example 1; the manufacture method was conducted analogously to the method outlined in example 1;

### Example N° 11

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 117.50 |
| Dibasic Calcium phosphate | 54.10 |
| Ethylcellulose | 10.00 |
| Talc | 5.50 |
| Magnesium Stearate | 2.75 |
| Ethanol | s.q. |
| **Total** | **250.00** |
| * CR means Controlled Released grade. | |

Second layer and coating are identical to example 1; the manufacture method was conducted analogously to the method outlined in example 1;

### Example N° 12

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 127.50 |
| Lactose | 19.85 |
| Microcrystalline Cellulose | 34.25 |
| Talc | 5.50 |
| Magnesium Stearate | 2.75 |
| Ethanol | s.q. |
| **Total** | **250.00** |
| * CR means Controlled Released grade. | |

Second layer and coating are identical to example 1; the manufacture method was conducted analogously to the method outlined in example 1;

### Example N° 13

### Core

| **A. First layer** | |
|---|---|
| **Layer pseudoephedrine** | **Mg/tablet** |
| Pseudoephedrine hydrochloride | 30.00 |
| Methylephedrine hydrochloride | 30.00 |
| Belladonna | 0.15 |
| Methocel K15M CR | 127.50 |
| Dibasic calcium phosphate | 54.10 |
| Talc | 5.50 |
| Magnesium Stearate | 2.75 |
| Ethanol | s.q. |
| **Total** | **250.00** |
| * CR means Controlled Released grade. | |

Second layer and coating are identical to example 1; the manufacture method was conducted analogously to the method outlined in example 1;

### Example N° 14

**a) Non-sustained release granules: 2 capsules (size 1)**

| | |
|---|---|
| Epinastine hydrochloride | 10.00 mg |
| Pseudoephedrine hydrochloride | 18.00 mg |
| Methylephedrine hydrochloride | 18.00 mg |
| Belladonna | 0.09 mg |
| Hydroxypropylcellulose | 3.50 mg |
| Sucrose | 499.41 mg |
| **Total** | **549.00 mg** |

**b) Sustained release granules: 2 capsules (size 1)**

| | |
|---|---|
| Pseudoephedrine hydrochloride | 42.00 mg |
| Methylephedrine hydrochloride | 42.00 mg |
| Belladonna | 0.21 mg |
| Hydroxypropylcellulose | 4.00 mg |
| Sucrose | 66.79 mg |
| Methaacrylic acid copolymer,type B | 40.60 mg |
| Glycerol esters of fatty acids | 3.10 mg |
| Talc | 1.30 mg |
| **Total** | **200 mg** |

### Capsulation

| | |
|---|---|
| non-sustained relaes granules | 549.00 mg |
| sustained relaes granules | 200.00 mg |
| Talc | 1.00 mg |
| Total | 750.00 mg |

### Method of Manufacture

### A. Non-sustained release granules

A1. Dissolve hydroxypropylcellulose in ethanol.
A2. Blend epinastine hydrochloride and pseudoephedrine hyrochloride, methylephedrine hydrochloride and belladonna in a suitable mixer and pulverize the powder mix.
A3. Produce spherical granules by spraying the solution prepared previously in step A1 over sucrose introducing the powder mix obtained from step A2.
A4. Dry and pass through granules from step A3 with suitable size screen to produce non-sustained release granules.

### B. Sustained release granules

B1. Dissolve hydroxypropylcellulose in ethanol.
B2. Blend pseudoephedrine hyrochloride, methylephedrine hydrochloride and belladonna in a suitable mixer.
B3. Produce spherical granules by spraying the solution prepared previously in step B1 over sucrose introducing the powder mix obtained from step B2.
B4. Dry and pass through granules from step B3 with suitable size screen
B5. Dissolve Methaacrylic acid copolymer,type B in ethanol and mix with glycerol esters of fatty acids and talc.
B6. Coat the granules obtained from step B4 with the solution prepared previously in step B5 to produce sustained release granules.

### C. Capusulation

C1. Mix non-sustained release granules and sustained release granules with talc.
C2. Fill the mixture obtained from step C1 into capsules

### Example N° 15

**a) Non-sustained release granules: 2 capsules (size 1)**

| | |
|---|---|
| Epinastine hydrochloride | 10.00 mg |
| Pseudoephedrine hydrochloride | 18.00 mg |
| Methylephedrine hydrochloride | 18.00 mg |
| Belladonna | 0.09 mg |
| Hydroxypropylcellulose | 3.50 mg |
| Sucrose | 499.41 mg |
| **Total** | **549.00 mg** |

**b) Sustained release granules: 2 capsules (size 1)**

| | |
|---|---|
| Pseudoephedrine hydrochloride | 42.00 mg |
| Methylephedrine hydrochloride | 42.00 mg |
| Belladonna | 0.21 mg |
| Hydroxypropylcellulose | 4.00 mg |
| Sucrose | 66.79 mg |
| Ethyllcellulose | 38.75 mg |
| Hydrooxypropylmethycellulose 2910 | 1.00 mg |
| Glycerol esters of fatty acids | 2.25 mg |
| Talc | 3.00 mg |
| **Total** | **200 mg** |

### Capsulation

| | |
|---|---|
| non-sustained relaes granules | 549.00 mg |
| sustained relaes granules | 200.00 mg |
| Talc | 1.00 mg |
| **Total** | 750.00 mg |

The manufacture method was conducted analogously to the method outlined in example 14.

### Example N° 16 Tablet

**a) Non-sustained release granules**

| | |
|---|---|
| Epinastine hydrochloride | 10.00 mg |
| Pseudoephedrine hydrochloride | 18.00 mg |
| Methylephedrine hydrochloride | 18.00 mg |
| Belladonna | 0.09 mg |
| Hydroxypropylcellulose | 12.5 mg |
| Microcrystalline cellulose | 178.91 mg |
| Lactose | 12.5 mg |
| **Total** | **250.00 mg** |

**b) Sustained release granules**

| | |
|---|---|
| Pseudoephedrine hydrochloride | 42.00 mg |
| Methylephedrine hydrochloride | 42.00 mg |
| Belladonna | 0.21 mg |
| Hydroxypropylcellulose | 4.00 mg |
| Sucrose | 66.79 mg |
| Methaacrylic acid copolymer,type B | 30.45mg |
| Magnesium stearate | 10.15 mg |
| Glycerol esters of fatty acids | 3.10 mg |
| Talc | 1.30 mg |
| **Total** | **200 mg** |

### Compression

| | |
|---|---|
| non-sustained relaes granules | 250.00 mg |
| sustained relaes granules | 200.00 mg |
| Microcrystalline cellulose | 126.00 mg |
| Croscarmellose sodium | 12.00 mg |
| Talc | 6.00 mg |
| Magnesium stearate | 6.00 mg |
| **Total** | **600.00 mg** |

### Method of Manufacture

### A. Non-sustained release granules

A1. Dissolve hydroxypropylcellulose in ethanol.
A2. Blend epinastine hydrochloride and pseudoephedrine hyrochloride, methylephedrine hydrochloride, belladonna, microcrystalline cellulose and lactose in a suitable mixer and knead the mixture with the solution from step A1.
A3. Dry and pass through granules obtained from step A2 with suitable size screen to produce non-sustained release granules

### B. Sustained release granules

B1. Dissolve hydroxypropylcellulose in ethanol.
B2. Blend pseudoephedrine hyrochloride ,methylephedrine hydrochloride and belladonna in a suitable mixer.
B3. Produce spherical granules by spraying the solution prepared previously in step B1 over sucrose introducing the powder mix obtained from step B2.
B4. Dry and pass through granules from step B3 with suitable size screen
B5. Dissolve Methaacrylic acid copolymer, type B in ethanol and mix with glycerol esters of fatty acids, magnesium stearate and talc.
B6. Coat the granules obtained from step B4 with the solution prepared previously in step B5 to produce sustained release granules.

### C. Compression:

C1. Mix non-sustained release granules and sustained release granules with microcrystalline cellulose, croscarmellose sodium, talc and magnesium stearate.
C2. Compress the mixture into a suitable tableting machine in suitable size tablets.

### Examples No° 17 to 32:

The same as examples No° 1 to 16 but the methylephrine is displaced by the same amount of pseudoephedrine, i.e. the amount of pseudoephedrine is doubled.

## Claims

1. Oral pharmaceutical compositions comprising as pharmaceutically active compounds a combination of a) an antihistaminic-effective amount of epinastine or a pharmaceutically acceptable salt thereof, b) an anticholinergic amount of Belladonna alkaloids or a pharmaceutically acceptable salt thereof and c) a decongestant-effective amount of pseudoephedrine or a pharmaceutically acceptable salt thereof optionally in combination with methylephrine or a pharmaceutically acceptable salt thereof and further comprising pharmaceutically acceptable carriers or excipients.

2. Oral pharmaceutical composition according to claim 1, **characterised in that** the Belladonna alkaloids comprise at least one alkaloid of the group being selected of atropin, L-(-)-hyoscyamin, L-(-)-hyoscin, N-Oxides of hyoscin and/or hyoscamin, atropamin, belladonnin and optionally nicotin, N-methylpyrrolin, N-methylpyrrolidin, pyridin, cuskhygrin.

3. Oral pharmaceutical composition according to claim 1 or 2, **characterised in that** all active ingredients are formulated for instant release.

4. Oral pharmaceutical composition according to any of claims 1 to 3, **characterised in that** epinastine or a pharmaceutically acceptable salt thereof is formulated for instant release and at least a portion of the other active ingredients, i.e. Belladonna alkaloids or a pharmaceutically acceptable salt thereof, pseudoephedrine or a pharmaceutically acceptable salt thereof and optionally methlyephrine, are formulated for sustained relesase.

5. Oral pharmaceutical composition according to claim 4, **characterised in that** the total amounts of Belladonna alkaloids or a pharmaceutically acceptable salt thereof, pseudoephedrine or a pharmaceutically acceptable salt thereof and optionally methlyephrine, are formulated for sustained relesase.

6. Oral pharmaceutical composition according to any of claims 1 to 5, **characterised in that** the formulation contains methylephrine or a pharmaceutically acceptable salt thereof.

7. Oral pharmaceutical composition according to any of claims 1 to 6, **characterised in that** the concentration range of epinastine or a pharmaceutically acceptable salt thereof is between 2 mg and 20 mg, the concentration range of the Belladonna alkaloids or salt pharmaceutically acceptable salt thereof is between 0.05 and 4.0 mg and the concentration range of pseudoephedrine plus methylephrine or the corresponding pharmaceutically acceptable salts thereof is between 5 and 240 mg.

8. Oral pharmaceutical composition according to claim 7, **characterised in that** the concentrations of pseudoephedrine and methylephrine are of the same amount.

9. Oral pharmaceutical composition according to any of claims 1 to 8, **characterised in that** it represents a bilayer tablet.

10. Bilayer tablet according to claim 9, wherein a first layer A, providing for the sustained release of Belladonna alkaloids, pseudoephedrine and optionally methylephrine or the corresponding pharmaceutical salts of the named active ingredients, comprises the amounts of theses compounds according to claim 1 and wherein a second layer B, providing for the immediate release of epinastine, comprises an antihistaminic effective amount of epinastine or a pharmaceutically acceptable salt thereof.

11. Bilayer tablet according to one of claims 9 or 10, **characterised in that** layer A comprises 60 mg pseudoephedrine hydrochloride, 60 mg methylephrine-hydrochloride and 0.3 mg Belladonna alkaloids and layer B comprises 10 mg epinastine-HCI or a tablet **in that** any of the amounts are diveded by 2.

12. Bilayer tablet according to any of claims 9 to 11, **characterised in that** it additionally contains a tablet coating C consisting of pharmaceutically acceptable excipients.

13. Bilayer tablet according to any of claims 9 to 12, **characterised in that** layer A comprises a decongestant effective amount of pseudoephedrine or a pharmaceutically acceptable salt thereof and methylephrine or a pharmaceutically acceptable salt thereof and an anticholinergic amount of the Belladonna alkaloids or a salt thereof in a matrix of a swellable hydrophilic polymer.

14. Capsule comprising an oral formulation according to one of claims 1 to 9.

15. Capsule according to claim 14, **characterised in that** the material of the capsules comprises a compound being selected from the group of chitosan and starch, grain powder, oligosaccharides, methacrylic acid-methylacrylate, methacrylic acid-ethylacrylate, hydroxypropylmethylcelluloseacetate, -succinate or -phthaleate, polyvinylalcohol, water-soluble non-toxic thermoplasts, hydroxypropylmethyl-cellulose, methylcellulose, hydroxypropylcellulose, hydroxypropylstarch, sodium alginate, gelatine and hard-gelatine.

16. Capsule according to claim 14 or 15, **characterised in that** the ingredients are formulated as sustained release and non-sustained release granules.

17. Capsule according to claim 16 in combination with claim 4 or 5.

18. Capsule according to claim 14, 15 or 16, **characterised in that** the non-sustained granules are coated by a water insoluble polymers, intestinally soluble polymers, paraffin waxes, higher alcohols, higher fatty acids and/or higher fatty acid esters.

19. Tablet comprising an oral formulation according to one of claims 1 to 9, **characterised in that** the ingredients are formulated as granules which are compressed to a tablet.

20. Tablet according claim 19, **characterised in that** the the ingredients are formulated as sustained release and non-sustained release granules.

21. Tablet according to claim 20 in combination with claim 4 or 5.

22. Tablet according to claim 19, 20 or 21, **characterised in that** that the non-sustained granules are coated by a water insoluble polymers, intestinally soluble polymers, paraffin waxes, higher alcohols, higher fatty acids and/or higher fatty acid esters.

23. Use of a pharmaceutical composition according to one of claims 1 to 9, a bilayer tablet according to any of claims 10 to 13, a capsule according to any of claims 14 to 18 or a tablet accroding to any of claims 19 to 21 for the treatment of saisonal allergic rhinitis, saisonal allergic conjunctivitis, allergic rhinitis, allergic congestion of the Eustachian tubes and / or other diseases from allergic origin deserving the administration of antihistamine and decongestant drugs, in the treatment of for instance common cold and in the symptomatic relief associated with cough, cold and flu symptoms.

24. Use according claim 22 for the treatment of seasonal allergic rhinitis and/or seasonal allergic conjunctivitis.
